# EUROPEAN PATENT SPECIFICATION

(11) **EP 1 091 707 B1**
(45) Date of publication and mention of the grant of the patent: **26.03.2003**
(21) Application number: 99933374.3
(22) Date of filing: 23.06.1999
(51) Int. Cl.: A61F 5/00

(54) **GASTRIC BAND**
MAGENBAND
CEINTURE GASTRIQUE

(30) Priority: 29.06.1998 US 106142
(43) Date of publication of application: 18.04.2001
(73) Proprietor: Obtech Medical AG, 6341 Baar (CH)
(72) Inventor: FORSELL, Peter, CH-6313 Menzingen (CH); JAKOBSSON, Arne, F-06600 Antibes (FR)
(74) Representative: Mercer, Christopher Paul
(86) International application number: PCT/SE99/01134
(87) International publication number: WO 00/000108

(56) References cited:
- EP-A- 0 611 561
- EP-A1- 0 769 282
- US-A- 3 875 928

## Description

The present invention relates to a food intake restriction device for forming a stoma opening in the stomach or esophagus of a patient, comprising a band including a closed tubing, means for forming a loop of the band defining a restriction opening, and fluid distribution means adapted to add fluid to the interior of the tubing to inflate the tubing and to withdraw fluid from the interior of the tubing to deflate the tubing, in order to adjust the size of the restriction opening. The band is intended for application around the stomach or esophagus to constrict the stomach or esophagus, so that the stoma opening is formed therein.

Such a food intake restriction device is known from EP 0611561 A1.

In the early 1980s, surgical procedures to treat overweight patients were often carried out by placing a band of a food intake restriction device around the stomach, which formed a restriction, thereby preventing food from passing downwards, or more correctly reducing the speed and the amount of food being eaten. After a few years of use of the new surgical method it became evident that it was very difficult to apply the band with an appropriate tightness - if the band was too tight around the stomach, patients were affected by vomiting attacks. Alternatively, if the band was too loose, the opening between the upper and lower parts of the stomach became too large, resulting in the eating or the weight problems being unaffected. Unfortunately, therefore many of these operations resulted in failure.

The solution to this problem was to provide a band having an inflatable balloon on the inside thereof, like a blood pressure cuff. This balloon could be connected to an injection port, making it possible to change the inside diameter of the band after the operation. In this way, if after operation the band was found to be too tight, it was possible to drain off some fluid through the injection port. This procedure increased the opening of the band loop, resulting in a larger restriction between the upper and lower parts of the stomach. On the other hand, if patients did not lose weight, it was possible to inject fluid through the injection port, thereby narrowing the restriction between the upper and lower parts of the stomach (the "stoma diameter"). This operation was clearly better than the earlier method, but unfortunately this operation was not without problems. Namely, there existed three main difficulties.

First, the loop band had a tendency to dislocate downwards towards the lower part of the stomach. This could be prevented by suturing the lower part of the stomach to the upper part of the stomach (called "tunnelling") to prevent the band from dislocating downwards towards the major curvature of the stomach. However, sometimes these sutures ruptured, thus negatively affecting a desired long term weight loss.

The second difficulty, revealed by resent research, was that the upper part of the stomach rapidly increased in size, up to approximately ten times of its original size, resulting in less weight reduction.

The third difficulty was that the prior band tended to migrate through the stomach wall.

An object of the present invention is to provide a simple reliable substantially non-pressurised food intake restriction device for reducing the food intake of a patient.

Another object of the invention is to provide a food intake restriction device in which the band is designed to minimise the risk of the band migrating through the stomach wall.

These objects are obtained by a food intake restriction device of the kind initially stated, which is characterised in that the band is elastic such that with the band formed in the loop the band is longitudinally extensible and expandable outwardly relative to the loop by the fluid added to the interior of the tubing. As a result, the extensible band is able to yield longitudinally to permit accidental large pieces of food to pass through the restriction opening.

Furthermore, the elastic circumferential wall of the band is able to yield radially under the influences of dynamic movements of the stomach, *i.e.* the stomach has a certain freedom to move where it is in contact with the band, which significantly reduces the risk of the band injuring or migrating through the stomach wall. If the stomach wall were prevented from moving along the region where the band contacts the stomach, the stomach wall would became thinner over time, which dramatically increases the risk of the band penetrating the stomach wall. Thus, the elastic characteristics of the band of the food intake restriction device of the invention results in a more natural co-operation between the band and the stomach, *i.e.* the muscle of the stomach wall is allowed to move properly in a physiological sense.

To achieve the necessary adjustment of the size of the restriction opening, the tubing is simply designed broad enough to expand sufficiently from a flattened state radially inwardly of the loop. Besides, a broad tubing is beneficial with regard to the large surface area of the tubing that will contact the stomach.

The thickness of the circumferential wall of the tubing may suitably vary, preferably continuously, in the circumferential direction, so that two opposite axially extending portions of the circumferential wall differ in thickness. As a result, a relatively greater inward expansion of the tubing is achieved when the band inflates. The thinner one of said two opposite axially extending portions may suitably be designed to form a row of bulges along the band, when the tubing is at least partly filled with fluid. This gives the advantage that the formation of creases on the inner side of the tubing as the band is bent into a loop is avoided, which dramatically increases the lifetime of the band. Furthermore, the creases developed in the stomach wall along the band are distributed into the pockets formed between adjacent bulges which is beneficial in a physiological sense. As an alternative, such a row of bulges may also be designed on a tubing having uniform wall thickness.

Alternatively, or in combination with varying wall thickness, the same result is achieved by designing said two axially extending wall portions with different elasticity, whereby the more elastic portion is located on the inner side of the band loop.

Advantageously, one of said two opposite axially extending portions of the circumferential elastic wall, which is intended to form the inner side of the band loop, may be pre-tensioned.

The means for forming a loop of the band may comprise two separate locking elements stronger than the tubing secured to the tubing at opposite ends thereof. Alternatively, the locking elements may form integral parts of the tubing itself and be of the same material as the tubing. More specifically, the locking elements may be designed to be joined to each other by suturing, snap-lock connection, or the like.

The invention is described in more detail in the following with reference to the accompanying drawings, in which
Figure 1 is a perspective view, which schematically shows a reinforced food intake restriction device in place on the stomach and esophagus following surgical implantation,
Figure 2 presents a schematic perspective view of the device shown in Fig. 1,
Figure 3 is a view of a band of a first embodiment of the food intake restriction device according to the present invention,
Figure 4 is a cross-section along the line V-V in Fig. 3,
Figure 5 is a cross-section through a modification of the band shown in Fig. 3 and forming a loop,
Figure 6 is a cross-section through another modification of the band shown in Fig. 3 and forming a loop, and
Figures 7 and 8 are a front view and a side view, respectively, of a band of a second embodiment of the food intake restriction device according to the present invention.

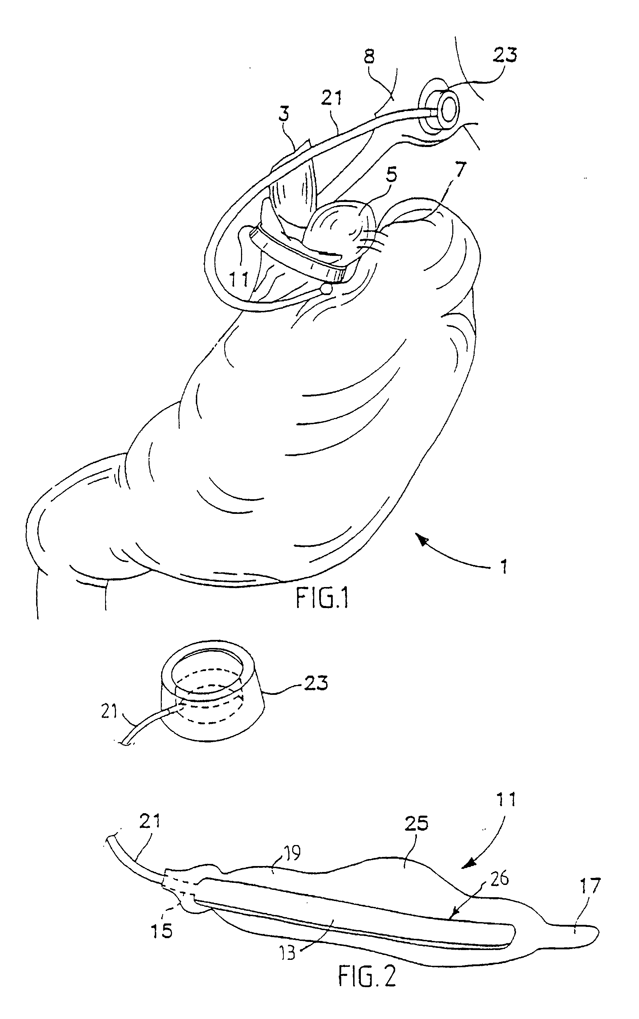

A reinforced food intake restriction device, which is subject of U.S. Patent Application No. 532,357 filed 1995, now U.S. Patent No. 5,771,903, will now be described with reference to Figs. 1 and 2. This device comprises a band 11, having a separate supporting elongated outer wall 13 of a substantially non-expansible and flexible material. Said wall 13 is preferably made of a reinforced plastic and/or silicone material and has such a flexibility that it could be bent to form a closed loop defining a restriction opening. The band 11 has a length enabling said closed loop of the band to be formed around the esophagus and/or stomach, and enabling an anterior upper part of the stomach wall to be pulled through said loop to form a small pouch 5 of the stomach. The ends 15, 17 of the outer wall 13 may be joined to each other, e.g. by suturing, by a snap-lock connection, or by any other suitable joining means.

The band 11 has an inner wall 19 made of an elastic, soft plastic and/or silicone material. Said inner wall 19 is glued or heat-sealed to the outer wall 13, thereby providing an expansible cavity between the walls 13, 19. The lateral extension of the elongated inner wall 19 in a fully inflated state is more than 20 mm and less than 40 mm, whereas the lateral extension of the outer wall 13 is about 13 mm. Fluid supply means are provided for adding fluid to and withdrawing fluid from said cavity to expand the flexible inner wall 19 to decrease the size of said restriction opening and deflate the flexible inner wall 19 to increase the size of said restriction opening. The fluid supply means comprise an injection port 23 and a flexible conduit 21 connecting the injection port 23 to said cavity. The injection port 23 is implanted in an easily accessible region on the patient, preferably it is placed subcutaneously against the lower part of the sternum 8, thereby providing a support for the injection port 23 during fluid injection. The flexible conduit 21, which is made of a tube of silicone rubber, has a length such that when the band 11 is applied on the stomach and the injection port 23 is implanted against the sternum 8, the conduit 21 extends downwardly from both the band 11 and the sternum 8 to form an open loop there between.

The inner wall of the band 19 may be inwardly expanded from adjacent the outer wall 13 to such an extent that when a band loop has been formed, the opening of the loop will be substantially obstructed. Normally, the unexpanded loop has an inner diameter of approximately 35 mm.

The band 11 varies in width along its long axis, thereby providing a support portion 25 in the middle of the band 11 with a greater area intended to rest against the esophagus 3 (or alternatively, on the posterior part of the esophagus-cardia junction, or on the posterior surface of the cardia). In this way the surface pressure against the esophagus 3 wall (or alternatively, on the posterior part of the esophagus-cardia junction, or on the posterior surface of the cardia) will be reduced, thereby diminishing the stress per unit area placed on the esophagus 3 wall (or alternatively, on the posterior part of the esophagus-cardia junction, or on the posterior surface of the cardia) by the band 11.

The wide range of adjustment of the cavity or the inner wall 19 is a very important feature of the band 11 for accomplishing a satisfying long-term result.

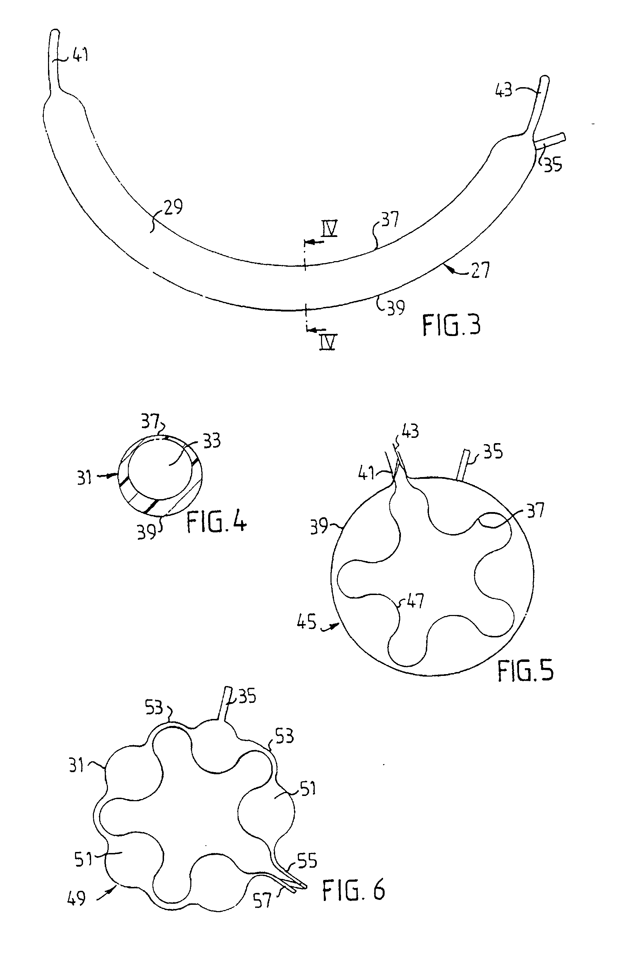

Fig. 3 shows a band 27 of an embodiment of the food intake restriction device of the present invention, for forming a stoma opening in the stomach or esophagus of a patient, having components similar to those of the device shown in Fig. 2, except that the band 27 is designed differently. The band 27 includes an elongated tubing 29 having a circumferential wall 31 of an elastic homogeneous material. The tubing 29 defines a closed cavity 33 with an inlet 35 for the supply of a fluid, preferably liquid. The circumferential wall 31 has a relatively thin axially extending portion 37 and a relatively thick axially extending portion 39 situated opposite thereof. The band 27 is provided with two interconnectible locking elements 41 and 43 for forming a loop of the band 27 defining a restriction opening. The locking elements 41,43 comprise end flaps formed as integral parts of the tubing 29 itself and are of the same material as the tubing 29.

As an alternative the circumferential wall 31 of the tubing 29 may be of a pliable material and not necessarily be elastic.

Fig. 5 shows a band 45 having like components as that of the band 27 shown in Fig. 3 except that the thin portion 37 of the circumferential wall 31 is designed differently. The two locking elements 41 and 43 of the band 45 are interconnected, so that the band 45 forms a loop with the thin portion 37 of the wall 31 extending innermost along said loop and consequently, with the thick portion 39 extending outermost along said loop. The thin portion 37 is designed to form a row of bulges 47 including at least three bulges 47, here there are five bulges 47, along the band 45. The bulges 47 are completely extended and adjacent bulges 47 are spaced apart from each other when the cavity 33 of the band 45 is filled with fluid.

Fig. 6 shows a band 49 having like components as that of the band 45 except that the circumferential wall 31 is uniformly thick and designed to form a number of interconnected but spaced apart spherical portions 51, here five portions 51, along the band 49.

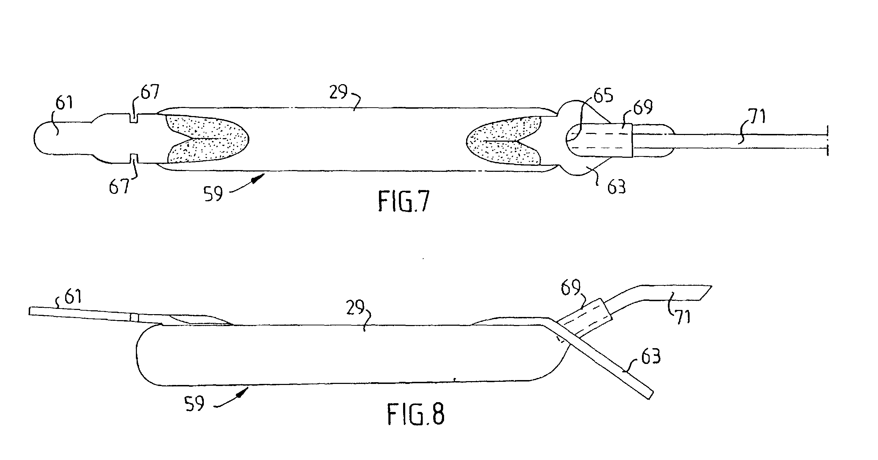

Fig. 7 and 8 show a band 59 of another embodiment of the food intake restriction device of the present invention having components similar to those of the device described above in connection with Figs. 3 and 4, except that said means for forming a loop of the band 59 (the locking elements 41,43 of band 27) are designed differently. Thus, two separate locking elements 61 and 63 stronger than the tubing 29 are secured, for instance by gluing, welding or the like, to the tubing 29 at opposite ends thereof, leaving a substantial longitudinal portion of the tubing 29 free between the end flaps 61,63. Each locking element 61,63 is secured to the same longitudinal side of the tubing 29, so that the entire opposite longitudinal side of the tubing 29 has a smooth surface suited for abutment against the stomach and/or esophagus. The locking element 61 is insertable into a hole 65 in the other locking element 63, so that the edge surrounding the hole 65 of the locking element 63 snaps into two lateral indentations 67 formed on the locking element 61. A sleeve 69 extends through the hole 65 and is glued on an edge surrounding an opening into the tubing 29. A pipe 71 is sealingly attached to the sleeve 69 for distributing fluid to and from the interior of the tubing 29.

## Claims

1. A food intake restriction device for forming a stoma opening in the stomach or esophagus of a patient, comprising a band (27,59) including a closed tubing (29), means (41,43;61,63) for forming a loop of the band defining a restriction opening, and fluid distribution means adapted to add fluid to the interior of the tubing to inflate the tubing and to withdraw fluid from the interior of the tubing to deflate the tubing, in order to adjust the size of the restriction opening, the band being intended for application around the stomach or esophagus to constrict the stomach or esophagus, so that the stoma opening is formed therein, **characterised in that** the band is elastic such that with the band formed in the loop the band is longitudinally extensible and expandable outwardly relative to the loop by the fluid added to the interior of the tubing.

2. A food intake restriction device according to claim 1, wherein the circumferential wall (31) of the tubing (29) is made of a homogeneous material.

3. A food intake restriction device according to claim 1, wherein the thickness of the circumferential wall (31) of the tubing (29) changes in the circumferential direction.

4. A food intake restriction device according to claim 3, wherein the thickness of the circumferential wall (31) of the tubing (29) changes continuously in the circumferential direction.

5. A food intake restriction device according to claim 3, wherein two opposite axially extending portions (37,39) of the circumferential wall (31) differ in thickness.

6. A food intake restriction device according to claim 5, wherein the thinner one (37) of the two opposite axially extending portions (37,39) of the circumferential wall (31) is designed to form a row of bulges (47) along the band (27,59), when the tubing (29) is at least partly filled with fluid.

7. A food intake restriction device according to claim 6, wherein the bulges (47) of the circumferential wall (31) are spaced apart from one another.

8. A food intake restriction device according to claim 1 or 2, wherein two opposite axially extending portions (37,39) of the circumferential wall (31) of the tubing (29) have different elasticity.

9. A food intake restriction device according to claim 1 or 2, wherein one of two opposite axially extending portions (37, 39) of the circumferential wall (31) of the tubing (29) is pretensioned.

10. A food intake restriction device according to claim 1, wherein one of two opposite axially extending portions (37,39) of the circumferential wall of the tubing (29) is designed to form a row of bulges (47) along the band (27,59), when the tubing is at least partly filled with fluid.

11. A food intake restriction device according to any of the preceding claims, wherein the means for forming a loop of the band comprise two separate locking elements (61,63) stronger than the tubing (29) secured to the tubing at opposite ends thereof.

12. A food intake restriction device according to any of the preceding claims, wherein the tubing comprises a non-reinforced tubing.

## Patentansprüche

1. Nahrungsaufnahme-Begrenzungs-Vorrichtung zum Ausbilden einer Stomaöffnung in dem Magen oder der Speiseröhre eines Patienten mit einem Band (27, 59), das ein geschlossenes Rohr (29), Mittel (41, 43; 61, 63) zum Bilden einer Schleife des Bands, wodurch eine Einengungsöffnung definiert wird, und Fluidverteilungsmitteln, die angepaßt sind, um Fluid zu dem Inneren des Rohrs zum Aufblähen des Rohrs hinzuzufügen und dem Inneren des Rohrs Fluid zum Entleeren des Rohrs zu entziehen, um die Größe der Einengungsöffnung anzupassen, wobei das Band für eine Anwendung um den Magen oder die Speiseröhre vorgesehen ist, um den Magen oder die Speiseröhre einzuschnüren, so daß die Stomaöffnung hierin ausgebildet wird, **dadurch gekennzeichnet, daß** das Band elastisch ist, so daß das in einer Schleife ausgebildete Band in Längsrichtung dehnbar und nach außen relativ zu der Schleife mittels des Hinzufügens des Fluids zu dem Inneren des Rohrs ausdehnbar ist.

2. Nahrungsaufnahme-Begrenzungs-Vorrichtung nach Anspruch 1, **dadurch gekennzeichnet, daß** die Umfangswand (31) des Rohrs (29) aus einem homogenen Material hergestellt ist.

3. Nahrungsaufnahme-Begrenzungs-Vorrichtung nach Anspruch 1, **dadurch gekennzeichnet, daß** die Dicke der Umfangswand (31) des Rohrs (29) sich in der Umfangsrichtung ändert.

4. Nahrungsaufnahme-Begrenzungs-Vorrichtung nach Anspruch 3, **dadurch gekennzeichnet, daß** die Dicke der Umfangswand (31) des Rohrs (29) sich kontinuierlich in der Umfangsrichtung ändert.

5. Nahrungsaufnahme-Begrenzungs-Vorrichtung nach Anspruch 3, **dadurch gekennzeichnet, daß** zwei entgegengesetzte, sich axial erstreckende Abschnitte (37, 39) der Umfangswand (31) sich in der Dicke unterscheiden.

6. Nahrungsaufnahme-Begrenzungs-Vorrichtung nach Anspruch 5, **dadurch gekennzeichnet, daß** der dünnere Abschnitt (37) der zwei entgegengesetzten, sich axial erstreckenden Abschnitte (37, 39) der Umfangswand (31) gestaltet ist, eine Reihe von Auswölbungen (47) entlang des Bands (27, 59) zu bilden, wenn das Rohr zumindest teilweise mit dem Fluid gefüllt ist.

7. Nahrungsaufnahme-Begrenzungs-Vorrichtung nach Anspruch 6, **dadurch gekennzeichnet, daß** die Auswölbungen (47) der Umfangswand (31) voneinander beabstandet sind.

8. Nahrungsaufnahme-Begrenzungs-Vorrichtung nach Anspruch 1 oder 2, **dadurch gekennzeichnet, daß** die zwei entgegengesetzten, sich axial erstreckenden Abschnitte (37, 39) der Umfangswand (31) des Rohrs (29) eine unterschiedliche Elastizität aufweisen.

9. Nahrungsaufnahme-Begrenzungs-Vorrichtung nach Anspruch 1 oder 2, **dadurch gekennzeichnet, daß** einer der zwei entgegengesetzten, sich axial erstreckenden Abschnitte (37, 39) der Umfangswand (31) des Rohrs (29) vorgespannt ist.

10. Nahrungsaufnahme-Begrenzungs-Vorrichtung nach Anspruch 1, **dadurch gekennzeichnet, daß** einer der zwei entgegengesetzten, sich axial erstreckenden Abschnitte (37, 39) der Umfangswand des Rohrs (29) konstruiert ist, eine Reihe Auswölbungen (47) entlang des Bands (27, 59) zu bilden, wenn das Rohr zumindest teilweise mit dem Fluid gefüllt ist.

11. Nahrungsaufnahme-Begrenzungs-Vorrichtung nach einem der vorangegangenen Ansprüche, **dadurch gekennzeichnet, daß** die Mittel zum Bilden einer Schleife des Bands zwei getrennte Verschlußelemente (61, 63) umfassen, die stärker als das Rohr (29) sind und an dem Rohr an entgegengesetzten Enden befestigt sind.

12. Nahrungsaufnahme-Begrenzungs-Vorrichtung nach einem der vorangegangenen Ansprüche, **dadurch gekennzeichnet, daß** das Rohr ein nicht verstärktes Rohr umfaßt.

## Revendications

1. Dispositif de restriction de la prise alimentaire pour former une ouverture de stomie dans l'estomac ou l'oesophage d'un patient, comportant une ceinture (27, 59) comprenant une tubulure fermée (29), des moyens (41, 43 ; 61, 63) pour former une boucle de la ceinture définissant une ouverture de restriction, et des moyens de distribution de fluide adaptés pour ajouter un fluide à l'intérieur de la tubulure, afin de gonfler la tubulure, et retirer un fluide de l'intérieur de la tubulure, afin de dégonfler la tubulure, de façon à ajuster la taille de l'ouverture de restriction, la ceinture étant destinée à être appliquée autour de l'estomac ou de l'oesophage pour créer une constriction de l'estomac ou de l'oesophage, de sorte que l'ouverture de stomie y soit formée, **caractérisé en ce que** la ceinture est élastique, de sorte qu'avec la ceinture formée dans la boucle, la ceinture est extensible longitudinalement et expansible vers l'extérieur par rapport à la boucle, par le fluide ajouté à l'intérieur de la tubulure.

2. Dispositif de restriction de la prise alimentaire selon la revendication 1, dans lequel la paroi circonférentielle (31) de la tubulure (29) est faite d'un matériau homogène.

3. Dispositif de restriction de la prise alimentaire selon la revendication 1, dans lequel l'épaisseur de la paroi circonférentielle (31) de la tubulure (29) varie dans le sens de la circonférence.

4. Dispositif de restriction de la prise alimentaire selon la revendication 3, dans lequel l'épaisseur de la paroi circonférentielle (31) de la tubulure (29) varie de manière continue dans le sens de la circonférence.

5. Dispositif de restriction de la prise alimentaire selon la revendication 3, dans lequel deux parties opposées s'étendant axialement (37, 39) de la paroi circonférentielle (31) ont une épaisseur différente.

6. Dispositif de restriction de la prise alimentaire selon la revendication 5, dans lequel la plus fine (37) des deux parties opposées s'étendant axialement (37, 39) de la paroi circonférentielle (31) est conçue pour former une rangée de protrusions (47) le long de la ceinture (27, 59) lorsque la tubulure (29) est au moins partiellement remplie de fluide.

7. Dispositif de restriction de la prise alimentaire selon la revendication 6, dans lequel les protrusions (47) de la paroi circonférentielle (31) sont espacées les unes des autres.

8. Dispositif de restriction de la prise alimentaire selon la revendication 1 ou 2, dans lequel deux parties opposées s'étendant axialement (37, 39) de la paroi circonférentielle (31) de la tubulure (29) ont une élasticité différente.

9. Dispositif de restriction de la prise alimentaire selon la revendication 1 ou 2, dans lequel l'une des deux parties opposées s'étendant axialement (37, 39) de la paroi circonférentielle (31) de la tubulure (29) est pré-tendue.

10. Dispositif de restriction de la prise alimentaire selon la revendication 1, dans lequel l'une des deux parties opposées s'étendant axialement (37, 39) de la paroi circonférentielle de la tubulure (29) est conçue pour former une rangée de protrusions (47) le long de la ceinture (27, 59) lorsque la tubulure est au moins partiellement remplie de fluide.

11. Dispositif de restriction de la prise alimentaire selon l'une quelconque des revendications précédentes, dans lequel les moyens pour former une boucle de la ceinture comportent deux éléments de fermeture distincts (61, 63) plus solides que la tubulure (29), fixés aux extrémités opposées de la tubulure.

12. Dispositif de restriction de la prise alimentaire selon l'une quelconque des revendications précédentes, dans lequel la tubulure comporte une tubulure sans renfort.
